(19) Europäisches Patentamt
European Patent Office
Office européen des brevets

(11) Publication number: 0 234 688
A1

(12) # EUROPEAN PATENT APPLICATION

(21) Application number: 87300188.7

(22) Date of filing: 09.01.87

(51) Int.Cl.[3]: C 07 D 333/22
C 07 D 333/24, C 07 D 333/32

(30) Priority: 15.01.86 GB 8600885

(43) Date of publication of application:
02.09.87 Bulletin 87/36

(84) Designated Contracting States:
BE CH DE FR GB IT LI NL

(71) Applicant: BEECHAM GROUP PLC
Beecham House Great West Road
Brentford Middlesex TW8 9BD(GB)

(71) Applicant: Wendstone Chemicals P.L.C.
Hanover House 14 Hanover Square
London W1R OBE(GB)

(72) Inventor: Ramage, Robert
26 Craigleith View, Ravelston
Edinburgh EH4 3JZ, Scotland(GB)

(72) Inventor: Johnstone, Alexander
63 Limpton Gate
Yarm-on-Tees, Cleveland(GB)

(74) Representative: Dayneswood, Trevor et al,
Beecham Pharmaceuticals Patent and Trade Mark
Department Biosciences Research Centre Great Burgh
Yew Tree Bottom Road
Epsom Surrey KT18 5XQ(GB)

(54) **Thiophene-3-carboxaldehyde derivatives, their preparation and their use in preparing 3-thienyl acetonitrile.**

(57) The novel compound 2,5-dihydrothiophene-3-carboxaldehyde (which exists in equilibrium with the further novel compound 4-hydroxy-tetrahydrothiophene-3-carboxaldehyde) may be prepared by the reaction of mercaptoacetaldehyde with acrolein. It may be used for the manufacture of 3-thienylacetonitrile, by reaction with a cyanide followed by elimination of an appropriate hydroxy compound.

EP 0 234 688 A1

TITLE MODIFIED
see front page



0234688

B1996

NOVEL COMPOUNDS AND PROCESSES

The present invention relates to novel chemical compounds, useful as chemical intermediates, to a process for their manufacture, and to their use.

More particularly, the present invention relates to the novel compounds 2,5-dihydrothiophene-3-carboxaldehyde and 4-hydroxy-tetrahydrothiophene-3-carboxaldehyde, and to a process for their manufacture. The novel compounds are useful as intermediates in the preparation of various 3-thienyl derivatives including, for example, thiophene-3-carboxaldehyde, thiophene-3-acetonitrile (3-thienylacetonitrile) and thiophene-3-malonic acid (3-thienyl-malonic acid).

3-thienylmalonic acid (formula I)

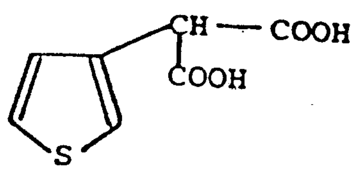

I

is an intermediate in the preparation of various pharmaceutical substances including, in particular, the commercially available semi-synthetic penicillins ticarcillin (formula II; see GB 1 004 670 - Beecham) and temocillin (formula III; see GB 1 538 052 - Beecham):

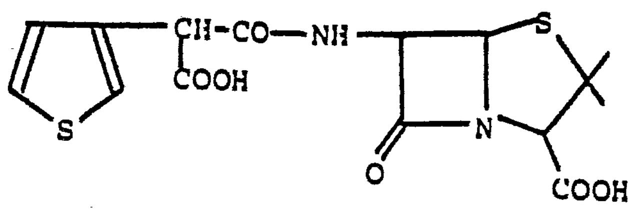

II

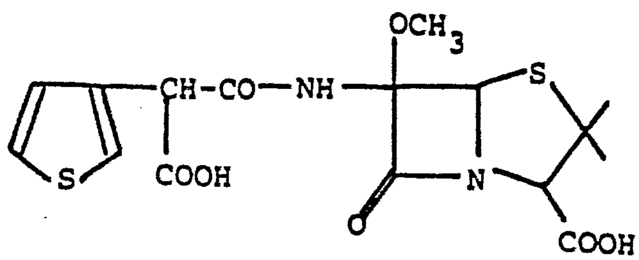

III

Numerous routes have been proposed for the manufacture of 3-thienylmalonic acid, and several such routes proceed through 3-thienylacetonitrile (formula IV)

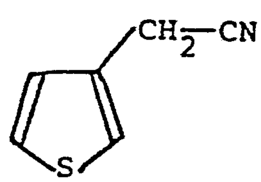

IV

which can then be converted into 3-thienylmalonic acid by various routes: (i) *via* ethyl 3-thienylacetate, diethyl 3-thienyl-oxaloacetate, and diethyl 3-thienylmalonic acid (GB 1 125 557, GB 1 359 991, and GB 1 359 992 - all Beecham); (ii) *via* a 3-thienylacetic acid ester (GB 1 426 557 - Beecham); or (iii) *via*

3-thienylcyanoacetate (EP 0 021 644 and EP 0 021 645 - both Beecham). Other routes to 3-thienyl-malonic acid have avoided 3-thienylacetonitrile (see, for example, EP 0 000 633 and EP 0 038 121 - both Beecham; GB 2 083 811 - Eastman Kodak; and GB 2 009 158 - OCE Andeno B.V.).

Various routes have also been proposed for preparing 3-thienylacetonitrile: (i) from 3-methylthiophene by bromination with N-bromosuccinimide and subsequent substitution with sodium cyanide (GB 1 125 557); (ii) by chlorination of thiophene to 2,5-dichlorothiophene, which is then chloromethylated, followed by substitution with sodium cyanide to give 2,5-dichloro-3-cyanomethylthiophene, which is converted to ethyl 3-thienylacetate and then to a 3-thienylmalonic acid diester (GB 1 359 991); or (iii) by reaction of methyl acrylate and methyl thioglycolate to give tetrahydrothiophen-3-one, followed by Knoevenagel condensation with cyanoacetic acid, and then dehydrogenation (GB 1 359 992). Those routes and their disadvantages are discussed in more detail in GB 2 009 158, which also describes other attempted processes for producing 3-thienylmalonic acid. Another method of producing 3-thienylacetonitrile, involving reaction of a 3-thienylmethyl halide with an alkali metal cyanide in the presence of ketones, water, and a phase transfer catalyst, has recently been described in EP 0 142 797 (BASF).

All such previously proposed routes to 3-thienylacetonitrile and to 3-thienylmalonic acid have various disadvantages. The novel compounds of the present invention render possible a new route to 3-thienylacetonitrile at lower cost from acyclic

starting materials and requiring only mild cyclisation conditions.

The compound 3-acetyl-2,5-dihydrothiophene has been described as an intermediate in the preparation of 3-acetyl-2,5-dihydrothiophene-1,1-dioxide as a stable precursor of 2-acetyl-1,3-butadiene. It was prepared by reaction of α-mercaptoaldehyde and methyl vinyl ketone in the presence of a base to give 3-acetyl-4-hydroxy-tetrahydrothiophene, which was isolated (48% yield) and then dehydrated in the presence of a base and methanesulphonyl chloride to give 3-acetyl-2,5-dihydrothiophene (approx. 60% yield; overall yield 29%) (J. F. Honek et al, Synthetic Communications 14(6), 483-491 (1984)).

The present invention now provides, in one aspect, the novel compound 2,5-dihydrothiophene-3-carboxaldehyde (formula V)

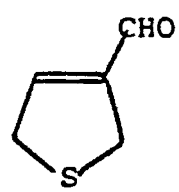

V

The novel compound 2,5-dihydrothiophene-3-carboxaldehyde according to the invention may be converted to 3-thienylacetonitrile as hereinafter described and may also be used in the preparation of other 3-thienyl derivatives.

The present invention also provides, in another aspect, a process for the manufacture of 2,5-dihydrothiophene-3-carboxaldehyde, which comprises reacting

α-mercaptoaldehyde (formula VI) or a dimeric (formula VII) or polymeric form thereof with acrolein (2-propenal; formula VIII):

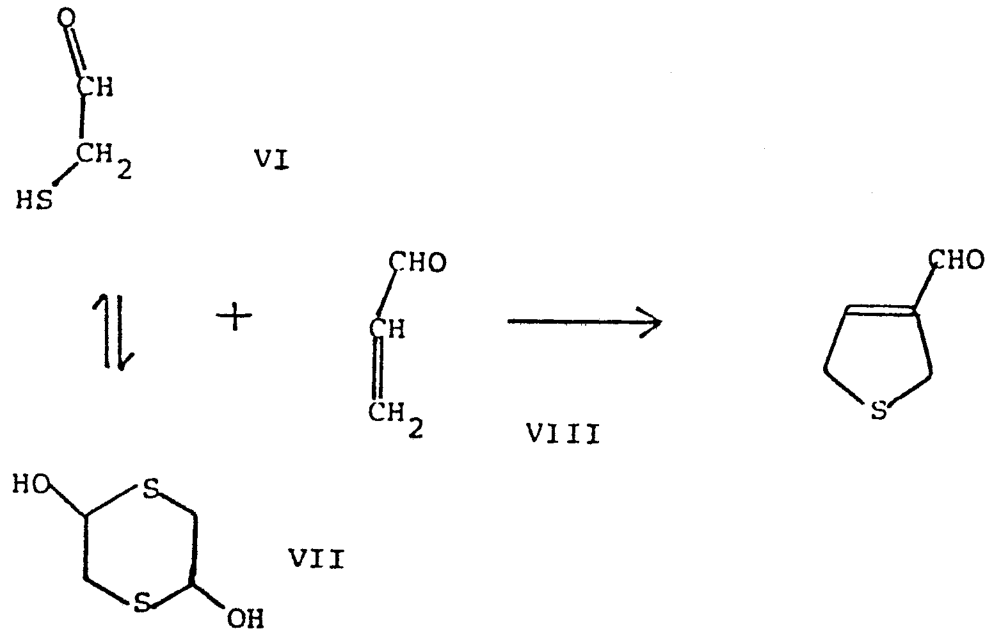

VI

VII

VIII

V

The present invention furthermore provides the novel compound 4-hydroxy-tetrahydrothiophene-3-carboxaldehyde (formula IX)

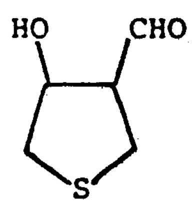

IX

which is an intermediate in the above-mentioned process according to the invention.

The starting material α-mercaptoaldehyde (VI) may suitably be used in the form of its commercially available dimer, p-dithiane-2,5-diol (VII). α-Mercaptoaldehyde may also exist in various polymeric,

or more particularly oligomeric, forms, and it may be used in such forms as well as in monomeric form. Moreover, the α-mercaptoaldehyde may be formed in situ by reaction of, for example, acetaldehyde and hydrogen sulphide (O.Hromatka and R.Habert, Monatsh 85, 1088-1096 (1954)), or diethylbromacetal and sodium sulphide to give the acetal (W.E.Parkham and H.Wynberg, 'Organic Syntheses', vol. IV, page 295).

The α-mercaptoaldehyde and the acrolein may suitably be used in approximately stoichiometric amounts, although either reagent may be used in a slight excess of, for example, up to 10 mole %.

The reaction of the α-mercaptoaldehyde with the acrolein may be effected in an aqueous or organic medium.

Examples of organic solvents suitable for use as the reaction medium include dichloroethane, dichloromethane, mesyl chloride, toluene and dimethylformamide.

Advantageously, the reaction is effected in a protic medium, preferably in an aqueous medium. Such an aqueous medium may comprise co-solvents additional to the water, and such co-solvents may suitably be protic or aprotic, polar, organic solvents, for example methanol, isopropanol or dimethylformamide. In such cases, the water should preferably be the major solvent component and, more preferably, should constitute at least 75% of the solvent. It is especially preferred that water should be the sole solvent.

In certain cases, especially when using an organic reaction medium, it may be advantageous to effect the reaction in the presence of small amounts (for example,

up to 10 mole %) of an acidic or basic catalyst, for example *p*-toluene-sulphonic acid or triethylamine. In other cases, however, particularly when using an aqueous reaction medium, there may be no need to include an acid or base.

The reaction may advantageously be effected at an elevated temperature (that is to say, above ambient temperature (20 - 25°C)), preferably at a temperature of at least 30°C, especially at least 50°C. The reaction may conveniently be effected under reflux.

The reaction to give the 2,5-dihydrothiophene-3-carboxaldehyde (V) proceeds through 4-hydroxy-tetrahydrothiophene-3-carboxaldehyde (IX). Under certain conditions those two compounds may be in mutual equilibrium. The equilibrium may, however, generally be forced toward either compound as desired, by appropriate control of the conditions (e.g. the pH of the reaction mixture) or by continuous removal of either compound from an equilibrating mixture. In particular the 2,5-dihydrothiophene-3-carboxaldehyde (V) may be obtained simply by separating or isolating that compound by conventional techniques.

The desired 2,5-dihydrothiophene-3-carboxaldehyde (V) may be separated out or isolated from the reaction mixture by conventional techniques, such as, for example, extraction into organic solvent, chromatography, vacuum distillation, crystallisation, or filtration. One convenient technique, which has the advantage of providing the desired compound in relatively pure form, is steam distillation of the reaction mixture, after which the desired product may be allowed to separate out from the distillate, or may be obtained by filtration of the distillate or by

extraction of the distillate with an organic solvent (for example, diethyl ether). Alternatively, the distillate could be used directly for subsequent reaction.

The 2,5-dihydrothiophene-3-carboxaldehyde (V) thus obtained is a useful intermediate in the preparation of 3-thienyl derivatives. In particular, according to another aspect of the present invention, it may be used to form 3-thienylacetonitrile (IV) by reaction with a cyanide, followed by elimination of an appropriate hydroxy compound.

The cyanide used for reaction with the 2,5-dihydrothiophene-3-carboxaldehyde (V) may be any suitable conventional cyanide source including, for example, hydrogen cyanide, organic cyanides, trimethylsilyl cyanide, and metal cyanides. Suitable metal cyanides include the alkali metal and alkaline earth metal cyanides, preferably sodium cyanide or potassium cyanide. The cyanide may, if desired, be produced *in situ*. The cyanide may conveniently be used in excess, for example in an excess of at least 50 mole % up to 100 mole % to 200 mole %.

The reaction of the aldehyde (V) with the cyanide may be effected using conventional reaction conditions for the reaction of a cyanide with an aldehyde. Conveniently, the reaction may be effected under acidic conditions, for example in the presence of glacial acetic acid, and at a depressed temperature (that is to say, below 20°C), advantageously at a temperature of from 0 to 15°C.

The reaction may, for example, be monitored by t.l.c. and, on completion, the reaction mixture may be

extracted with an organic solvent (for example, diethyl ether) to give 2,5-dihydrothiophene-3-carboxaldehyde cyanohydrin or a derivative thereof of the general formula X:

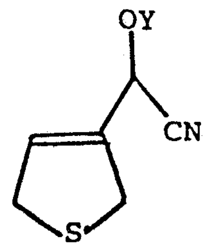

X

in which Y denotes a hydrogen atom or, for example, an acyl group (e.g. acetyl) or a trialkylsilyl group (e.g. trimethylsilyl).

When the reaction has been effected using hydrogen cyanide (or a source thereof) or a metal cyanide, the compound of the general formula X obtained will be the cyanohydrin (Y = H), whereas when it has been effected using, for example, a trialkylsilyl cyanide, the compound of the general formula X obtained will be the trialkylsilyl derivative. If desired, other derivatives may be obtained in conventional manner. Acyl derivatives (for example, the acetyl derivative (Y = $CH_3CO$)) may be obtained by reaction of the cyanohydrin (Y = H) with an anhydride (for example, acetic anhydride).

The cyanohydrin or derivative thereof of the general formula X may then subjected to an elimination reaction to eliminate the elements HOY (in which Y is defined as above), under conventional reaction conditions. In particular, when Y = H the elimination

reaction will be a water-elimination reaction. 1,4-elimination takes place to yield the desired 3-thienylacetonitrile IV.

The reaction is thought to proceed through 3-cyanomethylene-2,3-dihydrothiophene (formula XI):

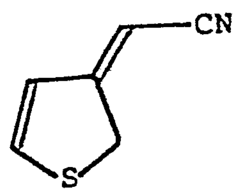

XI

The elimination may, for example, be effected by treatment with phosphoryl chloride, phosphoric acid, thionyl chloride, methanesulphonic anhydride, toluenesulphonyl chloride, or mesyl chloride, suitably in the presence of an organic or inorganic base (for example, triethylamine, dimethylethylamine, or N-methylpiperidine). The solvent (if any) and reaction conditions may suitably be those conventionally used for such elimination reactions. Suitable solvents include, for example, dichloromethane, toluene and dimethylformamide. The reaction may suitably be effected at an elevated temperature, for example, from 30°C up to reflux temperature.

The following Examples 1 and 2 illustrate the preparation of 2,5-dihydrothiophene-3-carboxaldehyde according to the invention; Example 3 illustrates its conversion to the cyanohydrin intermediate; and Examples 4 and 5 illustrate the conversion of the cyanohydrin to 3-thienylacetonitrile.

Example 1

2,5-Dihydrothiophene-3-carboxaldehyde

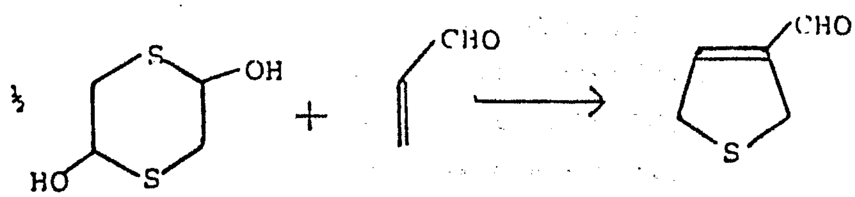

To a stirred suspension of *p*-dithianediol (75.1 g, 0.49 mole) in water (150ml) was added acrolein (65.2 g, 1.16 mole), over a period of 40 min, whilst the temperature was maintained at approximately 70$^{o}$C. When the addition was complete, the mixture was heated under reflux for 30 minutes. The apparatus was reassembled for steam distillation, which was carried out for 12 hours. The product separated out from the distillate (1.7 litres) as a crystalline solid on cooling to room temperature and it was filtered off and dried *in vacuo* at room temperature over $CaCl_2$. Yield 66.2g (58%); m.p. 45-46$^{o}$C. A further 12.8g (11%) of product (m.p. 41-43$^{o}$C) was extracted from the filtrates using diethyl ether. The product was characterised by I.R. and N.M.R.: $\nu_{max}$ (nujol mull) 1692 $cm^{-1}$; $\delta_H$ ($CDCl_3$, 80 MHz) 3.62 - 3.96 (4H, m, S($C\underline{H}_2$)$_2$), 6.76 - 6.86 (1H, m, C=C$\underline{H}$), 9.68 (1H, s, C$\underline{H}$O).

## Example 2

### 2,5-Dihydrothiophene-3-carboxaldehyde

Acrolein (4.70 g, 0.084 mole) and triethylamine (0.4 g, 0.004 mole) were added to a stirred slurry of dithianediol (6.08 g, 0.040 mole) in dichloroethane (200 ml) at 5°C under nitrogen. After 1 hour the mixture was warmed to 20°C, maintained for 1 hour, and *p*-toluene sulphonic acid monohydrate (1.52 g, 0.008 mole) was added. The temperature was raised to reflux, water being continuously removed *via* a reverse Dean and Stark trap. After 3 hours, analysis of the reaction mixture by HPLC showed 61% conversion to the required product.

Example 3

2,5-Dihydrothiophene-3-carboxaldehyde cyanohydrin

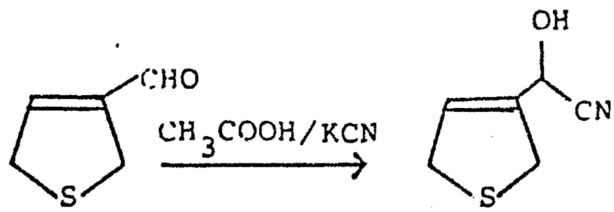


A stirred suspension of 2,5-dihydrothiophene-3-carboxaldehyde (25.5 g, 0.22 mole) in methanol (150 ml) was cooled to below 5°C and glacial acetic acid (42g, 0.7 mole) added. Solid potassium cyanide (37.9g, 0.57 mole) was then added to the mixture, while keeping the temperature below 12°C by cooling with an ice/water bath. The mixture was then stirred for a further 3 hours at approximately 5°C, after which time t.l.c. of the mixture indicated that all of the aldehyde had been converted to product. The mixture was transferred to a separating funnel with water (450 ml) and extracted with ether (2 x 200 ml). The combined extracts were washed with water, sodium bicarbonate solution and again with water. After drying over magnesium sulphate, the solvent was removed to yield the product as a straw coloured liquid (29.0 g, 96% yield). The cyanohydrin solidified during storage in the cold.

Example 4

Thiophene-3-acetonitrile

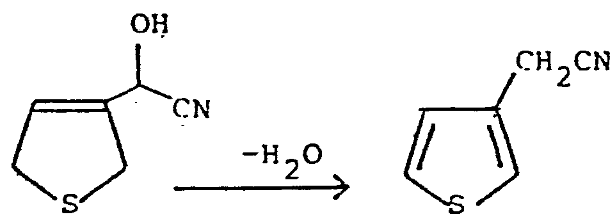


A solution of the cyanohydrin (9.5 g, 0.067 mole) from Example 3 in 50:50 toluene/pyridine (20 ml) was warmed to 50°C and a solution of phosphoryl chloride (14.0 g, 0.091 mole) in 50:50 toluene/pyridine (25 ml) added over a period of 15 min, whilst maintaining the temperature at approximately 60°C. At the end of the addition, the mixture was heated over a boiling water bath for 1 hour. The dark mixture was then poured into water (100 ml). The two layers which formed were separated, and the aqueous layer extracted with ether (50 ml). The combined organic layers were washed successively with 10% hydrochloric acid, water, sodium bicarbonate solution and water. After drying over magnesium sulphate, the solvent was removed to yield a dark red oil (6.5 g) of which 41% was the desired product (g.l.c. analysis at 160°C; 5 foot x 0.25 inch (1524 mm x 6.4 mm) glass column, packed with acid-washed dimethylchlorosilane-treated Chromosorb W coated with 5% w/w OV17, from Phase Separations Ltd; 'Chromosorb' is a trade mark). Estimated yield 2.7g (32%). The structure of the product was confirmed by comparison of its I.R. spectrum and g.l.c. retention time with those of an authentic sample of thiophene-3-acetonitrile. Pure material could be obtained by distillation at 80-82°C/1 mbar.

Example 5

Thiophene-3-acetonitrile

Dimethylethylamine (24 g, 0.33 mole) was added to a solution of the cyanohydrin from Example 3 (12.23 g, 0.076 mole) in dichloromethane (110 ml) at 0-5°C under nitrogen. Methanesulphonyl chloride (10.4 g, 0.009 mole) in dichloromethane (110 ml) was added dropwise over 1 hour with stirring, maintaining the temperature at 0-7°C. After about 6 hours at ice-bath temperature, the mixture was allowed to warm to about 20°C overnight. Excess amine and amine salts were removed by washing the solution with water (100 ml), 2N hydrochloric acid (2 x 120 ml), water (100 ml), 5% aqueous sodium bicarbonate solution (100 ml), and water (100 ml). The solution was dried over magnesium sulphate and the solvate removed to give an orange oil (10.174 g) containing 80.5% of the required product, equivalent to a yield of about 83%.

Assay of the product was carried out by HPLC (Waters 'Bondapack $C_{18}$', solvent 1:1 acetonitrile/water). Its structure was confirmed by N.M.R. and by conversion to 3-thienylmalonic acid.

0234688



**Claims** B1996

1. The compound 2,5-dihydrothiophene-3-carboxaldehyde of the formula

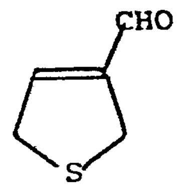

V

2. The compound 4-hydroxy-tetrahydrothiophene-3-carboxaldehyde of the formula

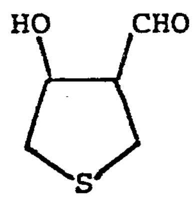

IX

3. A process for the manufacture of 2,5-dihydrothiophene-3-carboxaldehyde, which comprises reacting α-mercaptoaldehyde or a dimeric or polymeric form thereof with acrolein.

4. A process as claimed in claim 3, wherein the reaction is carried out in an aqueous medium.

5. A process for the preparation of 3-thienylacetonitrile, which comprises reacting 2,5-dihydrothiophene-3-carboxaldehyde with a cyanide to give a compound of the formula X

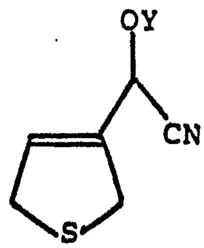

X.

in which Y denotes hydrogen or an organic group, and eliminating the elements HOY therefrom.

0234688

European Patent Office

# EUROPEAN SEARCH REPORT

Application number

EP 87300188.7

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | DE - A1 - 2 447 253 (BASF)<br>* Claims *<br>-- | 1,3 | C 07 D 333/22<br>C 07 D 333/24<br>C 07 D 333/32 |
| D,A | EP - A2 - 0 038 121 (BEECHAM)<br>* Claims *<br>-- | 5 | |
| D,A | EP - A2 - 0 142 797 (BASF)<br>* Claims *<br>---- | 5 | |
| | | | TECHNICAL FIELDS SEARCHED (Int. Cl.4) |
| | | | C 07 D 333/00 |

The present search report has been drawn up for all claims

| Place of search | Date of completion of the search | Examiner |
|---|---|---|
| VIENNA | 27-03-1987 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82